# EUROPÄISCHE PATENTSCHRIFT

(11) **EP 2 384 733 B1**
(45) Veröffentlichungstag und Bekanntmachung des Hinweises auf die Patenterteilung: **27.07.2016**
(21) Anmeldenummer: 10162250.4
(22) Anmeldetag: 07.05.2010
(51) Int. Cl.: A61K 6/00, A61K 6/02

(54) **Antimikrobielle Dentalwerkstoffe**
Antimicrobial dental materials
Matières dentaires antimicrobiennes

(43) Veröffentlichungstag der Anmeldung: 09.11.2011
(73) Patentinhaber: Ivoclar Vivadent AG, 9494 Schaan (LI)
(72) Erfinder: Salz, Ulrich, 88131, Lindau (DE); Rheinberger, Volker, 9490, Vaduz (LI); Rist, Kai, 6800, Feldkirch (AT)
(74) Vertreter: Uexküll & Stolberg

(56) Entgegenhaltungen:
- EP-A1- 2 113 237
- WO-A1-87/05501
- US-A- 4 206 215
- US-A1- 2006 275 339

## Beschreibung

Die vorliegende Erfindung betrifft Dentalwerkstoffe mit antimikrobieller Wirkung, die sich unter anderem als Füllungsmaterialien, Zemente, Versiegler, Materialien für temporäre Versorgungen, Beschichtungsmaterialien und Adhäsive eignen.

Die meisten zahnärztlichen Therapiemaßnahmen werden durch Besiedelung der Zahnhartsubstanz, der Randspalten zwischen der Zahnhartsubstanz und dentalen Restaurationen, von Restaurations- und prothetischen Materialien sowie des benachbarten Weichgewebes mit pathogenen Keimen bedingt. Besonders die Bakterien *Streptococcus mutans* und Lactobazillen produzieren Säuren als Stoffwechselprodukte, die, wie im Fall einer extensiven Karies, immense Hartgewebeschäden durch Gewebedecalzifizierung und enzymatischen Kollagenabbau verursachen. So ist es zum Beispiel bei einer Füllung eines Wurzelkanals mit einem bekannten inerten Füllungsmaterial nicht auszuschliessen, dass es durch im Kanal verbliebene Keime nach dem Füllen langsam zu einem entzündlichen Prozess kommt, welcher eine erneute Behandlung erforderlich macht, die dann sehr oft zum vollständigen Zahnverlust führt. Prothesenunverträglichkeiten sind häufig durch die Besiedelung der Prothesenoberflächen mit *Candida albicans,* einem Pilz der Candidagruppe, zurückzuführen.

Um pathogene, intraorale Mikroben zu unterdrücken, werden zum Beispiel zur Kariesprävention und -therapie Mundwässer, Zahnpasten und Gele angewendet, welche einen antimikrobiellen Wirkstoff wie z.B. Triclosan oder Chlorhexidin enthalten. Während Chlorhexidin eines der wirksamsten Mittel zur Bekämpfung von Plaque und Zahnfleischentzündungen ist, hat Triclosan den Vorteil, dass es nicht-ionisch und daher mit den meisten Bestandteilen von Mundpflegemitteln verträglich ist. Triclosan wird daher in den meisten kommerziell erhältlichen Produkten eingesetzt. Nachteilig bei diesen Applikationsformen ist die kurze Einwirkdauer. Diese macht eine langwierige, wiederholte Anwendung unabdingbar, um den gewünschten Therapieerfolg zu erzielen, was wiederum eine aufwendige Nachsorge bedingt. Bei schwer- oder gar unzugänglichen Besiedelungen mit pathogenen Keimen, wie tiefen Fissuren, Approximalräumen oder Randspalten, ist diese Therapieform ungeeignet. Bei der bakteriellen Besiedelung von Randspalten ist meist nur der Austausch der Füllung zielführend.

Um diesen destruktiven Therapiemassnahmen vorzubeugen, werden häufig antimikrobiell wirkende Dentalwerkstoffe eingesetzt. Die antimikrobielle Wirkung wird meist dadurch erzielt, dass dem Dentalwerkstoff antimikrobielle Wirkstoffe beigemischt werden. In der Regel werden die gleichen Wirkstoffe eingesetzt, die auch bei Mundwässern, Zahnpasten oder Gele verwendet werden. Problematisch ist, dass die physikalischen und chemischen Eigenschaften des Dentalwerkstoffs, wie seine mechanischen Eigenschaften oder das Aushärteverhalten, durch den Wirkstoffzusatz nicht beeinträchtigt werden dürfen. Auch muss eine Freisetzung des Wirkstoffs in einer wirksamen Menge und über einen längeren, klinisch relevanten Zeitraum sichergestellt werden.

In der WO 89/10736 wird ein Chlorhexidin enthaltender Dentalzement beschrieben, der in oralen Flüssigkeiten gut löslich ist und sich nach einer bis vier Wochen auflösen soll. Hierdurch soll eine kontrollierte Freisetzung des Wirkstoffs erzielt werden.

Die DE 198 13 686 A1 offenbart ein antimikrobiell wirksames Wurzelfüllmaterial, das Chlorhexidin und einen Jodophor enthält. Der Wirkstoff wird auf einen polymeren Träger aufgebracht.

In US 5,385,728 wird ein antimikrobielles, auf Phosphorsäure basierendes Ätzgel beschrieben, das Benzalkoniumchlorid in einem bevorzugten Konzentrationsbereich von 0.1% bis 4.0% Gew.-% enthält.

Die WO 99/20227 beschreibt ein Zahnbeschichtungsmaterial zur Kariesprävention, das als antimikrobiellen Wirkstoff Triclosan (2,4,4'-Trichlor-2'-hydroxydiphenylether) enthält.

In der US 2003/0220416 A1 werden härtbare dentale Restaurationsmaterialien beschrieben, die wasserunlösliche, nichtkationische antibakterielle Substanzen enthalten, vorzugsweise Triclosan.

Die EP 1 566 160 A2 offenbart die Verwendung von Extrakten aus Naturprodukten mit antimikrobieller Wirkung, wie zum Beispiel Grapefruitsamenextrakt oder Grüntee-Extrakt, zur Herstellung von Dentalwerkstoffen. Die Extrakte sind in Cyclodextrinen eingeschlossen.

Die WO 2005/058252 offenbart die Verwendung von elementarem Silber als antibakteriellem Wirkstoff für Dentalwerkstoffe, und die EP 1 374 830 A1 beschreibt eine dentale Zusammensetzung mit antimikrobiellen Eigenschaften, die silberhaltig Keramiken enthält.

Jeder dieser aufgeführten Wirkstoffe ist jedoch mit negativen Eigenschaften behaftet, die dessen Einsatz in Dentalwerkstoffen stark limitieren und die die klinische Tauglichkeit dieser Materialien in Frage stellen. Beispielsweise inhibieren Wirkstoffe wie Triclosan die radikalische Polymerisation, was die mechanischen Eigenschaften polymerisierbarer Dentalwerkstoffe beeinträchtigt und die einsetzbare Wirkstoffmenge beschränkt. Andere Wirkstoffe, wie z.B. Chlorhexidin, führen bei längerer oraler Anwendung zu einer deutlichen Schwarzfärbung von Zähnen, Zunge und Füllungen (Gjermo PJ, Dent Res, Spec Iss 1989; 68:1602-1608). Zudem führt Chlorhexidin durch seinen bitteren Geschmack zu Geschmacksirritationen und ist nicht lichtstabil. Bei Bestrahlung mit Sonnenlicht bildet sich das toxikologisch bedenkliche p-Chloranilin (Kohlbecker G, Dtsch Zahnärztl Z 1989; 44: 273-276). Silber führt durch die Bildung von schwarzem Silbersulfid oder gelb gefärbten Silberphosphat ebenfalls zu Verfärbungen in der Mundhöhle. Zudem ist das Wirkungsspektrum der genannten Wirkstoffe häufig nicht ausreichend.

Die EP 2 113 237 A1 offenbart mit Antiplaque-Wirkstoff(en) ausgestattete Dentalmaterialien, die als Wirkstoff ein molekulardispers verteiltes Octenidin-Salz oder Dequalinium-Salz enthalten. Diese Salze weist Anionen aus der Gruppe der Fettsäureanionen, Carbonsäureanionen, Alkyl- und Arylsulfonsäuren, Alkyl- und Arylsulfate auf. Es handelt sich hierbei um lipophile Anionen, die eine Mobilität/Migrationsfähigkeit der Octenidin-Salze in den Dentalmaterialien ermöglichen.

Der Erfindung liegt die Aufgabe zugrunde, Dentalwerkstoffe bereitzustellen, die sich durch eine hohe antimikrobielle Wirkung auszeichnen und die die genannten Nachteile nicht aufweisen.

Die Aufgabe wird erfindungsgemäß durch polymerisierbare Dentalwerkstoffe gelöst, die 0,1 bis 3,0 Gew.-% eines antimikrobiellen Wirkstoffs aus der Gruppe der Bis(4-Amino-1-pyridinium)salze gemäß der allgemeinen Formel (I) enthalten, in der A⁻ = Cl⁻, Br⁻, I⁻ oder F⁻ ist:

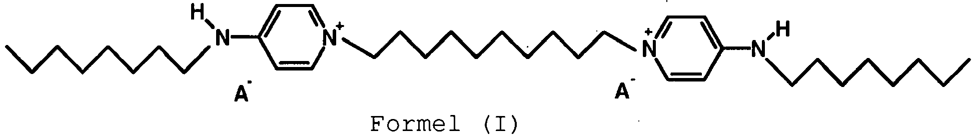

Die erfindungsgemäßen antimikrobiellen Wirkstoffe der Formel (I) lassen sich durch bekannte Syntheseverfahren wie z.B. in US 4,206,215 beschrieben herstellen.

Es wurde gefunden, dass die Wirkstoffe der Formel (I) eine zur Herstellung von Dentalwerkstoffen vorteilhafte Kombination von Eigenschaften aufweisen. Sie sind einerseits ausreichend lipophil, um mit organischen Monomeren verträglich zu sein, andererseits haben sie eine so große Wasserlöslichkeit, dass sie ihre antimikrobielle Wirkung voll entfalten können. Außerdem wurde gefunden, dass sie die radikalische Polymerisation nicht behindern.

Die erfindungsgemäßen Dentalwerkstoffe eignen sich insbesondere als Füllungsmaterialien und temporäre Füllungsmaterialien, z.B. für präparierte Kavitäten und Wurzelkanäle, als Unterfüllungsmaterialien, als Zemente und temporäre Zemente, beispielsweise zur Zementierung von indirekten Restaurationen, als Versiegler für Grübchen, Fissuren, Wurzelkanäle, Wurzel-, Dentin- und Schmelzoberflächen, als temporäre Versorgungsmaterialien, z.B. als Materialien für temporäre Kronen und Brücken, als Adhäsive, als Beschichtungsmaterialien, Ätzmittel, und Wurzelkanalsealer.

### Bevorzugte Dentalwerkstoffe sind:

Adhäsive, wie Schmelz/Dentinadhäsive, die vor der Adhäsivapplikation eine Substratkonditionierung mit einer Säure benötigen (Totaletch-Technik); selbstätzende Schmelz/Dentinadhäsive.

Temporäre Versorgungsmaterialien, wie temporäre Befestigungszemente auf Compositebasis; temporäre Befestigungszemente auf Polycarboxylat/Zinkoxidbasis; temporäre Füllungsmaterialien auf Compositebasis; temporäre Füllungsmaterialien auf Glasionomerbasis; temporäre Füllungsmaterialien auf Polycarboxylat/Zinkoxidbasis; temporäre Kronen- und Brückenmaterialien auf Composite- oder PMMA-Basis.

Zemente, wie Glasionomerzemente, Glasionomerhybridmaterialien, Polycarboxylatzemente.

Füllungsmaterialien, wie Compomerfüllungsmaterialien, Compositefüllungsmaterialien, Compositebefestigungsmaterialien, Bracketzemente.

Versiegler, wie Fissurenversiegler und Acrylatbeschichtungsmaterialien.

Die erfindungsgemäßen Dentalwerkstoffe enthalten 0,1 - 3,0 Gew.-%, bevorzugt 0,1 - 1,5 Gew.-% und besonders bevorzugt 0,1 - 0,6 Gew.-% des Wirkstoffs nach Formel (I). Alle Prozentangaben beziehen sich wenn nicht anders angegeben auf die Gesamtzusammensetzung der Dentalwerkstoffe.

Die polymerisierbaren Dentalwerkstoffe sind insbesondere Dentalwerkstoffe, die durch eine radikalische Polymerisation gehärtet werden können. Diese enthalten neben dem Wirkstoff nach Formel (I) mindestens ein polymerisierbares, ethylenisch ungesättigtes Monomer als Bindemittel, einen Initiator für die radikalische Polymerisation und können auch einen organischen und/oder anorganischen Füllstoff enthalten.

Die polymerisierbaren Dentalwerkstoffe eignen sich besonders
als Zahnfüllungsmaterialieh, Materialien für Inlays oder Onlays, Zahnzemente, als Verblendmaterialien für Kronen und Brücken, als Material für künstliche Zähne, Materialien für temporäre Versorgungen oder als sonstige Materialien für die prothetische, konservierende und präventive Zahnheilkunde,
als Schmelz/Dentinadhäsvie,
Coatingmaterial für Zahnfüllungen, Restaurationsmaterialien, Zahnprothese und natürliche Zähne.

Als anorganische Füllstoffe eignen sich besonders amorphe, kugelförmige Teilchen aus Siliciumdioxid und bis zu 20 Mol-% eines Oxids mindestens eines Elements der Gruppen I, II, III und IV des Periodensystems. Diese haben vorzugsweise einen Brechungsindex von 1,50 bis 1,58 und eine durchschnittliche Primärteilchengröße von 0,1 bis 1,0 µm.

Bevorzugte Füllstoffe sind weiterhin Quarz-, Glaskeramik- oder Glaspulver oder deren Mischungen. Diese weisen vorzugsweise ebenfalls einen Brechungsindex von 1,50 bis 1,58 und eine durchschnittliche Teilchengröße von 0,4 bis 5,0 µm auf.

Besonders bevorzugt werden Füllstoffmischungen verwendet, die mindestens zwei voneinander verschiedene Füllstoffe enthalten. Bevorzugt sind Füllstoffgemische, die als erste Füllstoffkomponente mindestens einen Füllstoff auf der Basis der obigen kugelförmiger Siliciumdioxidteilchen und als zweite Füllstoffkomponente mindestens ein Quarz-, Glaskeramik- und/oder Glaspulver enthalten.

Die amorphen, kugelförmigen Teilchen aus Siliciumdioxid sind ein amorphes, kugelförmiges Material auf der Basis von Siliciumdioxid, das daneben noch ein Oxid mindestens eines Metalles der Gruppen I, II, III und IV des Periodensystems enthält. Bevorzugt wird Strontium und/oder Zirkonoxid verwendet. Die durchschnittliche Primärteilchengröße liegt im Bereich von 0,1 bis 1,0 µm, insbesondere bei 0,15 bis 0,5 µm. Der Brechungsindex dieses anorganischen Füllstoffs liegt vorzugsweise zwischen 1,50 und 1,58, insbesondere zwischen 1,52 und 1,56. Ein besonders bevorzugter Wert ist 1,53 ± 0,01. Es können auch Mischungen unterschiedlicher Teilchen verwendet werden. Füllstoffe dieses Typs sind in der DE-PS 32 47 800 beschrieben. Die Füllstoffteilchen können auch gesintert als Mischung von Agglomeraten mit einer durchschnittlichen Teilchengröße von 1 bis 30 µm vorliegen.

Ebenso bevorzugte Füllstoffe sind Quarz-, Glaskeramik- und insbesondere Glaspulver. Die durchschnittliche Teilchengröße dieses anorganischen Füllstoffs soll zwischen 0,5 und 5,0 µm, insbesondere zwischen 1,0 und 2,0 µm und besonders bevorzugt zwischen 1,0 und 1,5 µm liegen, während der Brechungsindex Werte zwischen 1,50 und 1,58, insbesondere zwischen 1,52 und 1,56 aufweisen soll. Es können auch Füllstoffgemische eingesetzt werden. Bevorzugt werden erfindungsgemäß Ba-Silikatgläser mit einer mittleren Korngröße im Bereich von 1,1 bis 1,3 µm, sowie Sr-Silikatgläser mit einer mittleren Korngröße im Bereich von 1,1 bis 1,3 µm, sowie Li/Al-Silikatgläser mit einer mittleren Korngröße von 1,0 bis 1,6 µm verwendet. Solche Pulver lassen sich z.B. durch Feinmahlung mit einer RS-Ultrafeinstmühle der Firma Reimbold & Strich, Köln, erhalten.

Gegebenenfalls können neben der ersten und/oder zweiten Füllstoffkomponente noch weitere Füllstoffe zur Erzielung einer erhöhten Röntgenopazität eingesetzt werden, wobei deren mittlere Primärteilchengröße 5,0 µm nicht übersteigen sollte. Solche Füllstoffe sind z.B. in der DE-OS 35 02 594 beschrieben. Ein besonders bevorzugt verwendeter weiterer Füllstoff ist Ytterbiumtrifluorid.

Gegebenenfalls können zur Einstellung der Viskosität geringe Mengen an mikrofeiner, pyrogener oder nass gefällter Kieselsäure in den Dentalwerkstoff eingearbeitet werden, vorzugsweise jedoch höchstens 5 Gew.-%, bezogen auf den Dentalwerkstoff.

Die Gesamtfüllstoffmenge, bestehend aus den oben genannten Füllstoffen im erfindungsgemäßen Dentalwerkstoff liegt je nach Verwendungszweck zwischen 0 und 90 Gew.-%, vorzugsweise 2 bis 85 Gew.-% und besonders bevorzugt 10 bis 80 Gew.-% Vorzugsweise beträgt der Gewichtsanteil der ersten Füllstoffkomponente 5 - 60 %, insbesondere 10 - 30 %, der Anteil der zweiten Füllstoffkomponente 15 - 85 %, insbesondere 30 - 70 %, jeweils bezogen auf den Gesamtdentalwerkstoff.

Die anorganischen Füllstoffe sind bevorzugt silanisiert. Als Haftvermittler eignet sich z.B. α-Methacryloxypropyltrimethoxysilan. Die Menge des eingesetzten Haftvermittlers richtet sich nach der Art und BET-Oberfläche des Füllstoffes.

Als polymerisierbare organische Bindemittel eignen sich alle für einen Dentalwerkstoff brauchbaren Bindemittel, insbesondere monofunktionelle oder polyfunktionelle (Meth)acrylate oder (Meth)acrylamide, die allein oder in Mischungen eingesetzt werden können. Als Beispiele für diese Verbindungen kommen Methylmethacrylat, 2-Hydroxyethyl(meth)acrylat, 2-Hydroxypropyl-(meth)acrylat, Isobutyl(meth)acrylat, Cyclohexyl(meth)acrylat, Glycerindi(meth)acrylat, Tetraethylenglycoldi(meth)acrylat, Triethylenglycoldi(meth)acrylat, Diethylenglycoldi(meth)acrylat, Ethylenglycoldi(meth)acrylat, Polyethylenglycoldi(meth)acrylat, Butandioldi(meth)acrylat, Hexandioldi(meth)acrylat, Decandioldi-(meth)acrylat, Dodecandioldi(meth)acrylat, Bisphenol-A-di(meth)acrylat, Trimethylolpropantri(meth)acrylat, 2,2-Bis-4-(3-(meth)acryloxy-2-hydroxypropoxy)-phenylpropan (Bis-GMA) sowie die Reaktionsprodukte aus Isocyanaten, insbesondere Di- und/oder Triisocyanaten und OH-gruppenhaltigen (Meth)acrylaten in Frage. Beispiele dafür sind die Umsetzungsprodukte von 1 Mol Hexamethylendiisocyanat mit 2 Mol 2-Hydroxyethylenmethacrylat, von 1 Mol Tri(6-isocyanatohexyl)biuret mit 3 Mol 2-Hydroxyethylmethacrylat und von 1 Mol 2,2,4-Trimethylhexamethylendiisocyanat mit 2 Mol 2-Hydroxyethylmethacrylat, die im folgenden als Urethandimethacrylate bezeichnet werden.

N-mono- oder N-disubstitiuierte Acrylamide, wie z.B. N-Ethylacrylamid, N,N-Dimethylacrylamid, N-(2-Hydroxyethyl)-acrylamid oder N-Methyl-N-(2-hydroxyethyl)acrylamid, und N-monosubstituierte Methacrylamide, wie z.B. N-Ethylmethacrylamid oder N-(2-Hydroxyethyl)methacrylamid sowie außerdem N-Vinylpyrrolidon und Allylether. Diese Monomere sind bei Raumtemperatur flüssig und eignen sich daher auch als Verdünnermonomer.

Als Vernetzermonomere eignen sich Monomere mit zwei oder mehr radikalisch polymerisierbaren Gruppen, insbesondere vernetzende Pyrrolidone, wie z.B. 1,6-Bis-(3-vinyl-2-pyrrolidonyl)-hexan, oder kommerziell zugängliche Bisacrylamide wie Methylen- oder Ethylenbisacrylamid, Bis-(meth)acrylamide, wie z.B. N,N'-Diethyl-1,3-bis-(acrylamido)-propan, 1,3-Bis-(methacrylamido)-propan, 1,4-Bis-(acrylamido)-butan oder 1,4-Bis-(acryloyl)-piperazin, die durch Umsetzung aus den entsprechenden Diaminen mit (Meth)acrylsäurechlorid synthetisiert werden können.

Die erfindungsgemäßen Dentalwerkstoffe enthalten vorzugsweise mindestens ein Vernetzermonomer. Der Anteil der Vernetzermonomere im Dentalwerkstoff bewegt sich bevorzugt zwischen 10 und 95 Gew.-%, vorzugsweise 20 bis 75 Gew.-%.

Die erfindungsgemäßen Dentalwerkstoffe können auch ein radikalisch polymerisierbares, säuregruppenhaltiges Monomer enthalten. Säuregruppenhaltige Monomere werden im Folgenden auch als acide Monomere bezeichnet. Die Dentalwerkstoffe enthalten bevorzugt eine Mischung aus aciden und nicht aciden Monomeren. Bevorzugte Säuregruppen sind Carbonsäuregruppen, Phosphonsäuregruppen, Phosphatgruppen und/oder Sulfonsäuregruppen, wobei diese Gruppen in der Säureform oder in Form eines Esters vorliegen können. Bevorzugt sind Monomere mit Phosphonsäuregruppen oder Phosphatgruppen. Die Monomere können eine oder mehrere acide Gruppen aufweisen, bevorzugte sind Verbindungen mit 1 bis 2 aciden Gruppen.

Bevorzugte polymerisationsfähige Carbonsäuren sind Maleinsäure, Acrylsäure, Methacrylsäure, 2-(Hydroxymethyl)acrylsäure, 4-(Meth)acryloyloxyethyltrimellitsäure und das entsprechende Anhydrid, 10-Methacryloyloxydecylmalonsäure, N-(2-Hydroxy-3-methacryloyloxypropyl)-N-phenylglycin und 4-Vinylbenzoesäure.

Bevorzugte Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure 2-[4-(Dihydroxyphosphoryl)-2-oxa-butyl]-acrylsäure und 2-[2-Dihydroxyphosphoryl)-ethoxymethyl]-acrylsäure-2,4,6-trimethyl-phenylester.

Bevorzugte acide polymerisationsfähige Phosphorsäureester sind 2-Methacryloyloxypropylmono- und -dihydrogenphosphat, 2-Methacryloyloxyethylmono- und -dihydrogenphosphat, 2-Methacryloyloxyethyl-phenyl-hydrogenphosphat, Dipentaerythritolpentamethacryloyloxyphosphat, 10-Methacryloyloxydecyl-dihydrogenphosphat, Dipentaerythritolpentamethacryloyloxyphosphat, Phosphorsäuremono-(1-acryloyl-piperidin-4-yl)-ester, 6-(Methacrylamido)hexyldihydrogenphosphat und 1,3-Bis-(N-acryloyl-N-propylamino)-propan-2-yl-dihydrogenphosphat.

Bevorzugte polymerisationsfähige Sulfonsäuren sind Vinylsulfonsäure, 4-Vinylphenylsulfonsäure oder 3-(Methacrylamido)-propylsulfonsäure.

Der Anteil der aciden Monomere im Dentalwerkstoff bewegt sich zwischen 0 und 85 Gew.-%, vorzugsweise 0 bis 60 Gew.-%.

Außerdem können die erfindungsgemäßen Dentalwerkstoffe auch Lösungsmittel enthalten, vorzugsweise 0 bis 80 Gew.-%, besonders bevorzugt 0 bis 60 Gew.-% und ganz besonders bevorzugt 0 bis 40 Gew.-% Lösungsmittel.

Lösungsmittel werden hauptsächlich in Adhäsiven und Beschichtungsmaterialien eingesetzt. Bevorzugte Lösungsmittel sind Wasser, Methanol, Ethanol, Phenoxyethanol, Isopropanol, Ethylacetat, Aceton und Mischungen daraus.

Der Dentalwerkstoff kann je nach Art des verwendeten Initiators heiß, kalt oder durch Licht polymerisierbar sein. Als Initiatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch α,α'-Azo-bis-(isobutyroethylester), Benzpinakol und 2,2'-Dimethylbenzpinakol sind geeignet.

Als Initiatoren für die Photopolymerisation können z.B. Benzophenon und seine Derivate sowie Benzoin und seine Derivate verwendet werden. Weitere bevorzugte Photosensibilisatoren sind die α-Diketone wie 9,10-Phenanthrenchinon, Diacetyl, Furil, Anisil, 4,4'-Dichlorbenzil und 4,4'-Dialkoxybenzil. Campherchinon wird besonders bevorzugt verwendet. Die Verwendung der Photosensibilisatoren zusammen mit einem Reduktionsmittel ist bevorzugt. Beispiele für Reduktionsmittel sind Amine wie Cyanethylmethylanilin, Dimethylaminoethylmethacrylat, Triethylamin, Triethanolamin, N,N-Dimethylanilin, N-Methyldiphenylamin, N,N-Dimethyl-sym.-xylidin und N,N-3,5-Tetramethylanilin und 4-Dimethylaminobenzoesäureethylester. Des weiteren können Photoinitiatoren der Produktgruppe der Acylphosphinoxide, Bisacylphosphinoxide oder auch Acyl-Germaniumverbindungen eingesetzt werden.

Als Initiatoren für die Kaltpolymerisation werden Radikale liefernde Systeme, z.B. Benzoyl- bzw. Lauroylperoxid zusammen mit Aminen wie N,N-Dimethyl-sym.-xylidin oder N,N-Dimethyl-p-toluidin verwendet. Es können auch dual härtende Systeme zur Initiierung verwendet werden, z.B. Photoinitiatoren mit Aminen und Peroxiden. Als Photoinitiatoren kommen auch Mischungen aus UV-lichthärtenden und im Bereich des sichtbaren Lichts härtenden Initiatoren in Betracht.

Die Menge dieser Initiatoren im Dentalwerkstoff liegt üblicherweise zwischen 0,01 bis 5 Gew.-%, vorzugsweise 1 bis 3,5 Gew.-%.

Dem Dentalwerkstoff können ferner feinteilige Splitter- oder Perlpolymerisate einverleibt werden, die Homo- oder Copolymere der schon beschriebenen Vinylverbindungen sein können. Diese Homo- bzw. Copolymeren können ihrerseits mit den beschriebenen anorganischen Füllstoffen, auch röntgenopaken, gefüllt sein. Ferner kann der Dentalwerkstoff die üblichen Pigmentierungsmittel und Stabilisatoren enthalten.

Die erfindungsgemäßen Dentalwerkstoffe weisen bevorzugt die folgende Gesamtzusammensetzung auf:
0,1 - 3,0 Gew.-%, bevorzugt 0,1 - 1,5 Gew.-% und besonders bevorzugt 0,1 - 0,6 Gew.-% Wirkstoff gemäß Formel (I);
20 bis 95 Gew.-%, vorzugsweise 30 bis 90 Gew.-% und besonders bevorzugt 35 bis 80 Gew.-% Bindemittel;
0,1 bis 5 Gew.-%, vorzugsweise 0,3 bis 4,0 Gew.-% und besonders bevorzugt 0,5 bis 3,5 Gew.-% Initiator; und gegebenenfalls
0 bis 90 Gew.-%, vorzugsweise 2 bis 85 Gew.-% und besonders bevorzugt 10 bis 80 Gew.-% Füllstoff; und gegebenenfalls
0 bis 80 Gew.-%, besonders bevorzugt 0 bis 60 Gew.-% und ganz besonders bevorzugt 0 bis 40 Gew.-% Lösungsmittel.

Die erfindungsgemäßen Dentalwerkstoffe eignen sich beispielsweise zur Verhinderung von Zahnschädeh, die durch *Streptococcus mutans* und / oder Lactobazillen hervorgerufen werden oder auch zur Verhinderung von Prothesenunverträglichkeiten, die durch *Candida albicans* hervorgerufen werden.

Die Erfindung wird im Folgenden anhand von Beispielen und Abbildungen näher erläutert.

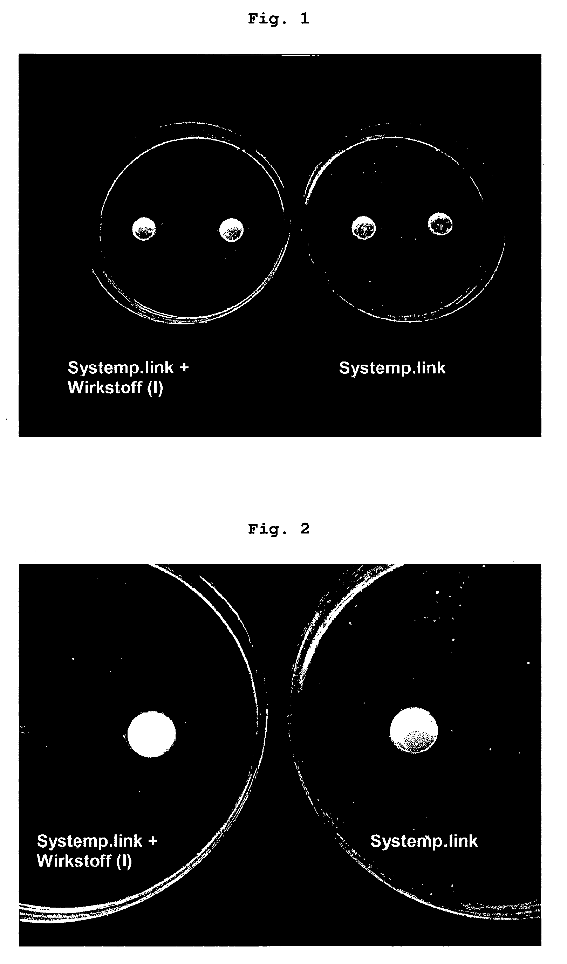

Figur 1 zeigt Hemmhöfe, die sich auf mit *S. mutans* beimpften Agarplatten um Plättchen aus einem erfindungsgemäßen Dentalwerkstoff bilden. Im Vergleich dazu bilden sich um Plättchen aus Dentalwerkstoff ohne Wirkstoff keine Hemmhöfe. Der Test zeigt, dass Wirkstoffe der Formel (I) aus polymerisierten Dentalwerkstoffen in einer wirksamen Menge freigesetzt werden.
Figur 2 zeigt einen vergrößerten Ausschnitt aus Figur 1.

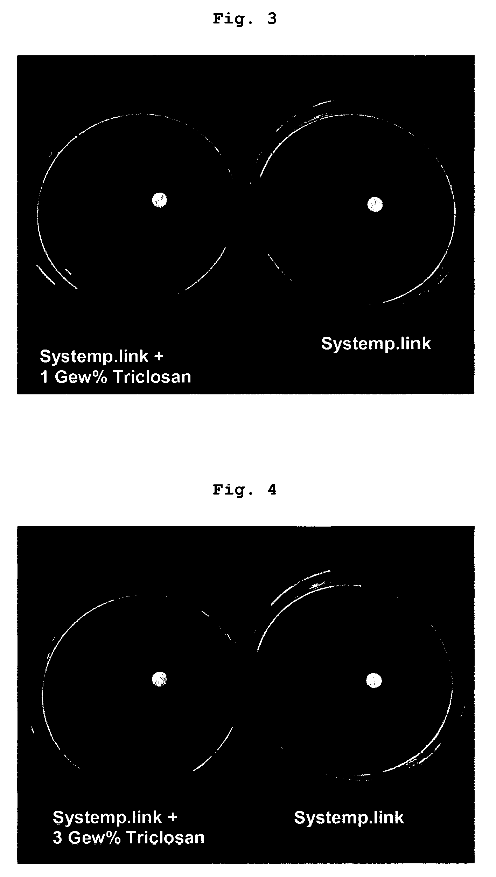

Figur 3 zeigt Plättchen eines ausgehärteten Vergleichsbeispiels das Triclosan in einer Konzentration von 1,0 Gew.-% enthält auf einer mit *S. mutans* beimpften Agarplatte. Im Vergleich zu dem Plättchen aus einem erfindungsgemäßen Dentalwerkstoff bilden sich um Plättchen aus Dentalwerkstoff mit Triclosan als Wirkstoff keine Hemmhöfe.
Figur 4 zeigt Plättchen eines ausgehärteten Vergleichsbeispiels das Triclosan in einer Konzentration von 3,0 Gew.-% enthält auf einer mit *S. mutans* beimpften Agarplatte. Im Vergleich zu dem Plättchen aus einem erfindungsgemäßen Dentalwerkstoff bilden sich um Plättchen aus einem Dentalwerkstoff mit Triclosan als Wirkstoff keine Hemmhöfe.

### Beispiel 1:

### Antimikrobielle Schmelz/Dentinadhäsive

Für die Untersuchung des Einflusses des antimikrobiellen Wirkstoffs nach Formel (I) auf die Dentin- und Schmelzhaftung von Adhäsivformulierungen wurden Mischungen der folgenden Zusammensetzung hergestellt:

### Beispiel 1a: Antimikrobielles Schmelz/Dentinadhäsiv für die Totaletch-Technik

(i) 17,1 Gew.-% des Vernetzers Glycerindimethacrylat (GDMA);
(ii) 69 Gew.-% des hydrophilen Monomers 2-Hydroxyethylmethacrylat (HEMA);
(iii) 12 Gew.-% Ethanol;
(iv) 1,0 Gew.-% des antimikrobiellen Wirkstoffs nach Formel (I);
(v) 0,5 Gew.-% Ethyl-p-dimethylaminobenzoat;
(vi) 0,4 Gew.-% Campherchinon.

Bei dem nicht-antimikrobiellen Vergleichsadhäsiv wurde der antimikrobielle Wirkstoff durch die entsprechende Menge an GDMA ersetzt.

### Haftwertuntersuchungen (Scherhaftfestigkeit)

Für die Dentin- bzw Schmelz-Haftmessungen wurden in Harz (Bühler Castolite-Resin) gegossene Rinderzähne verwendet. Zuerst wurde der eingegossene Rinderzahn approximal mit Schleifpapier der Körnung P500 bis zum Dentin bzw. Schmelz abgeschliffen. Nach kurzem Feinschliff mit Schleifpapier der Körnung P1000 und gründlichem Abwaschen mit Wasser wurde der Zahn für die Prüfkörperherstellung präpariert.

### Prüfkörperherstellung

Auf die Dentin- bzw. Schmelzoberfläche wurde Phosphorsäure-Ätzgel (Emailpräparator® GS, Ivoclar Vivadent AG) aufgetragen und nach einer Einwirkzeit von 30 Sekunden mit Wasser abgewaschen. Die so konditionierte Oberfläche wurde mit einem Papiertuch leicht trocken getupft und anschliessend die Adhäsivformulierung aufgetragen. Nach einer Einwirkzeit von 10 Sekunden wurde die Adhäsivschicht durch Bestrahlen mit einer Polymerisationslampe (Astralis® 7) für 20 Sekunden polymerisiert. Der so präparierte Zahn wurde in einer Scherhaftform gespannt, ein Füllungscomposite mittels einer runden Delrinform von 4 mm Durchmesser in zwei Schichten (insgesamt max. 3 mm) aufgetragen und jeweils für 40 Sekunden mit einer Polymersationslampe (Astralis® 7 der Ivoclar Vivadent AG; >500 mW/cm²) belichtet. Der so hergestellte Prüfkörper wurde entformt und sofort in Wasser gelegt. Nach Lagerung der Prüfkörper bei 37°C und 24h wurde die Scherhaftfestigkeit mit einer Universalprüfmaschine (Zwick Z010) und einer Prüfkörpervorrichtung (Guillotine) bei einer Geschwindigkeit von 0,8 mm/Min. ermittelt. Die erzielten Haftwerte sind in Tabelle 1 aufgeführt.

### Beispiel 1b: Selbstätzendes, antimikrobielles Schmelz/Dentinadhäsiv

(i) 20 Gew.-% des Säuremonomers Methacryloyloxydecyldihydrogenphosphat (MDP);
(ii) 20 Gew.-% des Säuremonomers 4-Methacryloyloxyethyltrimellitat Anhydrid (4-META);
(iii) 28,1 Gew.-% des Vernetzermonomers Urethandimethacrylat (UDMA);
(iv) 29,3 Gew.-% einer 1:1 Aceton/Wassermischung;
(v) 1,0 Gew.-% des antimikrobiellen Wirkstoffs der Formel (I);
(vi) 0,4 Gew.-% des Photoinitiators Campherchinon;
(vii) 0,5 Gew.-% des Coinitiators Ethyl-p-dimethylaminobenzoat;
(viii) 0,7 Gew.-% des Photoinitiators 2,4,6-Trimethylbenzoyldiphenylphosphinoxid.

Bei dem nicht-antimikrobiellen Vergleichsadhäsiv wurde der antimikrobielle Wirkstoff durch die entsprechende Menge an UDMA ersetzt.

Die Haftwertbestimmung dieses selbstätzenden antimikrobiellen Schmelz/Dentinadhäsivs wurde analog zu Beispiel 1a durchgeführt, jedoch mit dem Unterschied, dass das Substrat vor dem Aufbringen des Adhäsivs nicht mit Phosphorsäure konditioniert wurde. Die erzielten Haftwerte sind in Tabelle 1 aufgeführt.

Wie aus der Tabelle 1 zu entnehmen ist, wirkt sich der Zusatz des antimikrobiellen Wirkstoffes der Formel (I) nicht negativ auf die Dentin- bzw. Schmelzhafteigenschaften der Adhäsive aus.

**Tabelle 1: Dentin- und Schmelzhaftwerte der Adhäsive aus den Beispielen 1a - 1c**

| **Adhäsiv** | **Dentinhaftung** | **Schmelzhaftung** |
|---|---|---|
| | **(MPa)** | **(MPa)** |
| Bsp. 1a | 17,9 ± 3,3 | 19,8 ± 4,7 |
| Vergleich zu Bsp. 1a*) | 19,1 ± 5,2 | 20,0 ± 4,6 |
| Bsp. 1b | 23,2 ± 3,2 | 20,1 ± 4,1 |
| Vergleich zu Bsp. 1b*) | 21,9 ± 5,2 | 19,8 ± 5,2 |

| | | |
|---|---|---|
| *) enthält keinen antimikrobiellen Wirkstoff | | |

### Beispiel 2:

### Antimikrobielles Bondingmaterial

Durch Mischen der Komponenten wurde die folgende Zusammensetzung hergestellt:
(i) 50,1 Gew.-% bis-GMA;
(ii) 49,1 Gew.-% TEGDMA;
(iii) 0,1 Gew.-% des antimikrobiellen Wirkstoffs der Formel (I);
(iv) 0,3 Gew.-% Campherchinon;
(v) 0,4 Gew.-% des Coinitiators Genocure EPD;

Nach 20 s Belichtung mit einer dentalen Polymerisationslampe (Bluephase® C8, Ivoclar Vivadent AG) härtete das Bondingmaterial vollständig aus.

### Beispiel 3:

### Antimikrobieller temporärer Befestigungszement auf PolycarboxylatlZinkoxidbasis

In die Initiatorpaste des kommerziell erhältlichen temporären Befestigungszements auf Polycarboxylat/Zinkoxidbasis Systemp.cem (Ivoclar Vivadent AG) wurden 0,3 Gew-% bzw. 3,0 Gew.-% des antimikrobiellen Wirkstoffs der Formel (I) eingearbeitet. Anschliessend wurden die Verarbeitungszeit (VZ), Abbindezeit (AZ) und Shore D-Härte bestimmt. Die Ergebnisse sind in Tabelle 2 zusammengefasst.

**Tabelle 2: Eigenschaften von temporären Zementen auf Polycarboxylat/Zinkoxidbasis**

| **Zement** | **Shore-D Härte** | **VZ (s)** | **AZ (s)** |
|---|---|---|---|
| Systemp.cem*) | 56 | 115 | 208 |
| Systemp.cem + 0,3 Gew.-% I | 57 | 108 | 199 |
| Systemp.cem + 3,0 Gew.-% I | 59 | 105 | 187 |

| | | | |
|---|---|---|---|
| *) Vergleich | | | |

Wie Tabelle 2 zeigt, hat der Zusatz von 0,3 bzw. 3,0 Gew.-% von Wirkstoff (I) auf die Aushärtung von Systemp.cem keinen signifikanten, negativen Einfluss.

System.cem ist ein Zweikomponenten-Befestigungszement für die temporäre Befestigung von provisorischen Kronen und Brücken auf Zinkoxid-Polycarboxylatbasis. Die Basispaste besteht aus einer Mischung von Zinkoxid, Glycerin und Wasser, die Katpaste besteht aus einer Mischung von SiO2-Partikeln, Polyacrylsäure, Wasser und Olivenöl.

### Beispiel 4:

### Antimikrobieller temporärer Befestigungszement auf Compositebasis

In die Basispaste des kommerziell erhältlichen temporären Befestigungszements auf Compositebasis Systemp.link (Ivoclar Vivadent AG) wurden 0,3 Gew-% bzw. 3,0 Gew.-% des antimikrobiellen Wirkstoffs der Formel (I) eingearbeitet. Anschliessend wurden die Verarbeitungszeit (VZ), Abbindezeit (AZ) und Shore D-Härte bestimmt. Die Ergebnisse sind in Tabelle 3 zusammengefasst.

**Tabelle 3: Eigenschaften von temporären Zementen auf Compositebasis**

| **Zement** | **Shore-D Härte** | **VZ (s)** | **AZ (s)** |
|---|---|---|---|
| Systemp.link*) | 40 | 258 | 117 |
| Systemp.link + 0,3 Gew.-% I | 40 | 258 | 119 |
| Systemp.link + 3,0 Gew.-% I | 42 | 262 | 116 |

| | | | |
|---|---|---|---|
| *) Vergleich | | | |

Wie Tabelle 3 zeigt, hat der Zusatz von 0,3 bzw. 3,0 Gew.-% von Wirkstoff (I) auf die Aushärtung von Systemp.link keinen signifikanten, negativen Einfluss.

System.link ist ein dualhärtender Zweikomponenten-Befestigungszement für die temporäre Befestigung von provisorischen Kronen und Brücken auf Compositebasis. Die Basispaste besteht aus einer Mischung von SiO₂-Partikeln, einem Splitterpolymerisat, einer Monomermischung und einem tertiären aromatischen Amin. Die Katpaste besteht aus einer Mischung von SiO2-Partikeln, einem Splitterpolymerisat, einer Monomermischung, Dibenzoylperoxid und einem Campherchinon/Amin Photoinitiatorsystem.

### Beispiel 5:

### Antimikrobielles temporäres Füllungsmaterial

In das kommerziell erhältliche temporäre Füllungsmaterial auf Compositebasis Fermit (Ivoclar Vivadent AG) wurden 0,2 Gew-% bzw. 0,5 Gew.-% des antimikrobiellen Wirkstoffs der Formel (I) eingearbeitet. Nach 20 s Belichtung mit einer dentalen Polymerisationslampe (Bluephase®, Ivoclar Vivadent AG) härtete das Material vollständig aus. Anschliessend wurden die Shore D-Härte bestimmt (Ergebnisse siehe Tabelle 4).

**Tabelle 4: Shore-D-Härte von temporären Füllungsmaterialien**

| **Füllungsmaterial** | **Shore-D Härte** |
|---|---|
| Fermit*) | 43 |
| Fermit + 0,2 Gew.-% I | 45 |
| Fermit + 0,5 Gew.-% I | 44 |

| | |
|---|---|
| *) Vergleich | |

Wie Tabelle 4 zeigt, hat der Zusatz von 0,2 bzw. 0,5 Gew.-% von Wirkstoff (I) auf die Aushärtung von Systemp.link keinen signifikanten, negativen Einfluss.

Fermit ist ein temporäres Einkomponenten-Füllungsmaterial, das mit Blaulicht ausgehärtet werden kann. Die Zusammensetzung besteht aus einer Mischung von SiO₂-Partikeln, einem Splitterpolymerisat, einer Monomermischung und einem Campherchinon/Amin Photoinitiatorsystem.

### Beispiel 6:

### Antimikrobielles Coatingmaterial

(i) 12,0 Gew.-% Methylmethacrylat;
(ii) 66,7 Gew.-% Di-Pentaerythritoltriacrylat;
(iii) 0,1 Gew.-% des antimikrobiellen Wirkstoffs der Formel (I) ;
(iv) 7,9 Gew.-% SiO₂-Nanopartikel;
(v) 11,5 Gew.-% Ethanol;
(vi) 1,0 Gew.-% 2,4,6-Trimethylbenzoyldiphenylphosphinoxid;
(vii) 0,3 Gew.-% Campherchinon; und
(viii) 0,5 Gew.-% des Coinitiators Ethyl-p-dimethylaminobenzoat.

Nach 20 s Belichtung mit einer dentalen Polymerisationslampe (Bluephase®, Ivoclar Vivadent AG) härtete das Coatingmaterial vollständig ohne Sauerstoffinhibierung aus.

### Beispiel 7:

### Antimikrobielles Flowable-Füllungsmaterial

Es wurde ein Compositefüllungsmaterial mit folgender Zusammensetzung hergestellt:

| **Komponente** | **Gew.-%** |
|---|---|
| Bindemittel¹⁾ | 16.8% |
| Füllstoff²⁾ | 48.5% |
| Präpolymer | 34% |
| Additive, Katalysatoren und Stabilisatoren | 0.7% |
| Pigmente | <0.1% |

| | |
|---|---|
| ¹⁾ Bis-GMA, Urethandimethacrylat, ethoxiliertes Bis-EMA ²⁾ Bariumglasfüller, Ytterbiumtrifluorid, Mischoxid | |

In das Compositefüllungsmaterial wurden 3,0 Gew-% bzw. 5,0 Gew.-% des antimikrobiellen Wirkstoffs der Formel (I) eingearbeitet. Nach 20 s Belichtung mit einer dentalen Polymerisationslampe (Bluephase®, Ivoclar Vivadent AG) wurde die Durchhärtungstiefe bestimmt. Als Vergleich wurden auch die antimikrobiellen Wirkstoffe Triclosan und Chlorhexidin Diacetat in einer Menge von 3,0 Gew-% bzw. 5,0 Gew.-% in das Compositematerial eingearbeitet und die Durchhärtungstiefe bestimmt (Ergebnisse siehe Tabelle 5).

Wie Tabelle 5 zeigt, hat der Zusatz von Wirkstoff (I) auf die Durchhärtungstiefe des Compositematerials keinen signifikanten, negativen Einfluss. Hingegen verringert sich die Durchhärtungstiefe des Compositematerials durch den Zusatz von Triclosan beziehungsweise Chlorhexidin Diacetat drastisch.

**Tabelle 5: Durchhärtungstiefe von lichthärtenden Compositefüllungsmaterialien**

| **Compositefüllungsmaterial** | **Durchhärtungdtiefe (mm)** |
|---|---|
| Compositematerial*) | 4,12 |
| Compositematerial + 3,0 Gew.-% Triclosan | 3,04 |
| Compositematerial + 5,0 Gew.-% Triclosan | 2,48 |
| Compositematerial + 3,0 Gew.-% Chlorhexidin Diacetat | 3,83 |
| Compositematerial + 5,0 Gew.-% Chlorhexidin Diacetat | 3,17 |
| Compositematerial + 3,0 Gew.-% I | 4,13 |
| Compositematerial + 5,0 Gew.-% I | 4,09 |

| | |
|---|---|
| *) Vergleich | |

### Beispiel 8:

### Einfluss von Triclosan und des Wirkstoffs der Formel (I) auf die radikalische Polymerisation

Zu der Basispaste des kommerziell erhältlichen temporären Befestigungszements auf Compositebasis Systemp.link (Ivoclar Vivadent AG), der radikalisch aushärtet, wurden 3,0 Gew-% Triclosan bzw. 3,0 Gew.-% des antimikrobiellen Wirkstoffs der Formel (I) eingearbeitet. Anschliessend wurden die Verarbeitungszeit (VZ), Abbindezeit (AZ) und Shore D-Härte bei chemischer Aushärtung bestimmt. Die Ergebnisse sind in Tabelle 6 zusammengefaßt.

**Tabelle 6: Eigenschaften von temporären Befestigungszementen**

| **Temporärer Zement** | **Shore-D Härte** | **VZ (s)** | **AZ (s)** |
|---|---|---|---|
| Systemp.link*) | 40 | 258 | 117 |
| Systemp.link + 3,0 Gew.-% Triclosan*) | nicht polym. | nicht polym. | nicht polym. |
| Systemp.link + 3,0 Gew.-% I | 41 | 260 | 115 |

| | | | |
|---|---|---|---|
| *) Vergleich | | | |

Wie Tabelle 6 zeigt, hat der Zusatz von Wirkstoff (I) auf die Aushärtung von Systemp.link keinen signifikanten, negativen Einfluss. Hingegen wird durch den Zusatz von Triclosan die chemische Aushärtung von Systemp.link vollständig inhibiert.

### Beispiel 9:

### Bestimmung der antimikrobiellen Wirkung eines temporären Zements auf Compositebasis mit dem Wirkstoff nach Formel (I)

Zur Bestimmung der antimikrobiellen Wirkung eines temporären Dentalzements mit dem Wirkstoff der Formel (I) wurden in den kommerziell erhältlichen Zement Systemp.link (Ivoclar Vivadent AG) 3,0 Gew.-% des Wirkstoffs der Formel (I) eingearbeitet. Anschliessend wurden runde Prüfkörper mit einem Durchmesser von 10 mm und einer Dicke von 2 mm mit dem entsprechenden Material zu Plättchen geformt und dann gehärtet. Als Referenz dienten Prüfkörper aus Systemp.link ohne Wirkstoff sowie mit 1,0 und 3,0 Gew.-% Triclosan. Danach wurde die antimikrobielle Wirkung auf den Bakterienstamm *Streptococcus mutans* untersucht. Dazu wurde der Agarplatten Hemmhoftest angewendet.

| | |
|---|---|
| Bakterienstamm: | *S. mutans* DSM20723 |
| Kultivierungsmedium: | BHI Broth |
| Agar: | BHI |

Auf Agarplatten wurden 100 µl unverdünnte *S. mutans* Kulturlösung ausplattiert. Anschliessend wurden die Prüfkörper auf die Agarplatten aufgebracht. Nach 24h Lagerung bei 37°C/5% CO₂ wurden die Platten ausgewertet.

Wie in Figuren 1 und 2 ersichtlich ist, sind bei den mit dem Wirkstoff der Formel (I) versetzten Systemp.link-Plättchen (lichtgehärtet) deutliche Hemmhöfe zu erkennen. Die Referenzprobe zeigte hingegen keine Inhibierung des Bakterienwachstums (keine Hemmhofbildung). Überraschenderweise bewirkt auch der Zusatz von 1,0 bzw. 3,0 Gew.-% Triclosan keine Hemmhofbildung (Figuren 3 und 4). Der Test zeigt, dass die erfindungsgemäßen Dentalwerkstoffe den Wirkstoff der Formel (I) in einer wirksamen Menge freisetzen.

## Patentansprüche

1. Polymerisierbarer Dentalwerkstoff, **dadurch gekennzeichnet, dass** er 0,1 - 3,0 Gew.-% eines antimikrobiellen Wirkstoffs gemäß der allgemeinen Formel (I) enthält, 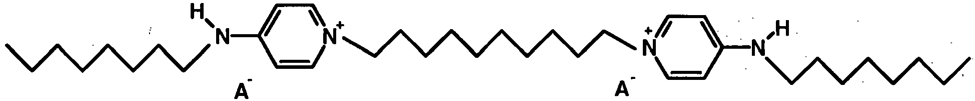 in der
A⁻ = Cl⁻, Br⁻, I⁻ oder F⁻ ist.

2. Dentalwerkstoff nach Anspruch 1, der 0,1 - 1,5 Gew.-% und bevorzugt 0,1 - 0,6 Gew.-% des Wirkstoffs gemäß Formel (I) enthält.

3. Dentalwerkstoff nach einem der vorhergehenden Ansprüche, der zusätzlich mindestens ein polymerisierbares, ethylenisch ungesättigtes Monomer als Bindemittel und einen Initiator für die radikalische Polymerisation enthält.

4. Dentalwerkstoff nach einem der vorhergehenden Ansprüche, der zusätzlich einen organischen und/oder anorganischen Füllstoff oder ein Füllstoffgemisch enthält.

5. Dentalwerkstoff nach Anspruch 3, der als Bindemittel mindestens ein monofunktionelles oder polyfunktionelles (Meth)acrylat oder (Meth)acrylamid oder eine Mischung davon enthält.

6. Dentalwerkstoff nach Anspruch 3 oder 5, der als Bindemittel eine Mischung aus aciden und nicht aciden Monomeren enthält.

7. Dentalwerkstoff nach einem der vorhergehenden Ansprüche, der zusätzlich ein Lösungsmittel enthält, vorzugsweise Wasser, Methanol, Ethanol, Phenoxyethanol, Isopropanol, Ethylacetat, Aceton oder eine Mischung von zwei oder mehr der genannten Lösungsmittel.

8. Dentalwerkstoff nach einem der Ansprüche 1-3 oder 5-6, der
0,1 - 1,5 Gew.-% und bevorzugt 0,1 - 0,6 Gew.-% Wirkstoff gemäß Formel (I);
20 bis 95 Gew.-%, vorzugsweise 30 bis 90 Gew.-% und besonders bevorzugt 35 bis 80 Gew.-% Bindemittel;
0,1 bis 5 Gew.-%, vorzugsweise 0,3 bis 4,0 Gew.-% und besonders bevorzugt 0,5 bis 3,5 Gew.-% Initiator; und gegebenenfalls
0 bis 90 Gew.-%, vorzugsweise 2 bis 85 Gew.-% und besonders bevorzugt 10 bis 80 Gew.-% Füllstoff; und gegebenenfalls
0 bis 80 Gew.-%, besonders bevorzugt 0 bis 60 Gew.-% und ganz besonders bevorzugt 0 bis 40 Gew.-% Lösungsmittel enthält.

9. Dentalwerkstoff nach einem der vorhergehenden Ansprüche zur Verwendung als Zahnfüllungsmaterial, Zahnzement, Versiegler oder temporäres Versorgungsmaterial.

10. Dentalwerkstoff nach einem der Anspruche 1 bis 8 zur Verwendung als Schmelz/Dentinadhäsiv.

11. Dentalwerkstoff nach einem der Anspruche 1 bis 8 zur Verwendung als Coatingmaterial für Zahnfüllungen, Restaurationsmaterialien, Zahnprothesen und natürliche Zähne.

12. Dentalwerkstoff nach einem der Anspruche 1 bis 8 zur Verhinderung von Zahnschäden, die durch *Streptococcus mutans* und / oder Lactobazillen hervorgerufen werden.

13. Dentalwerkstoff nach einem der Anspruche 1 bis 8 zur Verhinderung von Prothesenunverträglichkeiten, die durch *Candida albicans* hervorgerufen werden.

14. Verwendung eines Wirkstoffs gemäß Formel (I) zur Herstellung eines polymerisierbaren Dental werkstoffs gemäß Anspruch 1.

## Claims

1. Polymerisable dental material, **characterised in that** it comprises 0.1 -
3.0 wt % of an antimicrobial active substance of the general Formula (I), 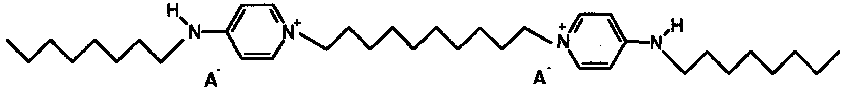 in which
A⁻ = Cl⁻, Br⁻, I⁻ or F⁻.

2. Dental material according to claim 1 comprising 0.1 - 1.5 wt % and preferably 0.1 - 0.6 wt % of the active substance of Formula (I).

3. Dental material according to one of the preceding claims additionally comprising at least one polymerisable, ethylenically unsaturated monomer as a binding agent and an initiator for radical polymerisation.

4. Dental material according to one of the preceding claims additionally comprising an organic and/or inorganic filler or a mixture of fillers.

5. Dental material according to claim 3 comprising at least one monofunctional or polyfunctional (meth)acrylate or (meth)acrylamide or a mixture thereof as the binding agent.

6. Dental material according to claim 3 or 5 comprising a mixture of acidic and non-acidic monomers as the binding agent.

7. Dental material according to one of the preceding claims additionally comprising a solvent, preferably water, methanol, ethanol, phenoxyethanol, isopropanol, ethyl acetate, acetone or a mixture of two or more of the cited solvents.

8. Dental material according to one of claims 1 - 3 or 5 - 6 comprising 0.1 - 1.5 wt % and preferably 0.1 - 0.6 wt % of the active substance of Formula (I);
20 to 95 wt %, preferably 30 to 90 wt % and particularly preferably 35 to 80 wt % of binding agent;
0.1 to 5 wt %, preferably 0.3 to 4.0 wt % and particularly preferably 0.5 to 3.5 wt % of initiator; and optionally
0 to 90 wt %, preferably 2 to 85 wt % and particularly preferably 10 to 80 wt % of filler; and optionally
0 to 80 wt %, preferably 0 to 60 wt % and quite particularly preferably 0 to 40 wt % solvent.

9. Dental material according to one of the preceding claims for use as a tooth filling material, tooth cement, sealing material or material for temporary care.

10. Dental material according to one of claims 1 to 8 for use as an enamel/dentine adhesive.

11. Dental material according to one of claims 1 to 8 for use as a coating material for tooth fillings, restoration materials, dentures and natural teeth.

12. Dental material according to one of claims 1 to 8 for the prevention of damage to teeth caused by *Streptococcus mutans* and / or Lactobacilli.

13. Dental material according to one of claims 1 to 8 for the prevention of prosthesis incompatibilities caused by *Candida albicans.*

14. Use of an active substance of Formula (I) for manufacturing a polymerisable dental material according to claim 1.

## Revendications

1. Matériau dentaire polymérisable, **caractérisé en ce qu'**il contient de 0,1 à 3,0 % en poids d'un agent antimicrobien de formule générale (I) : 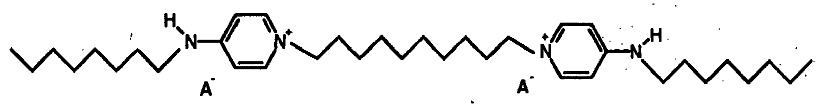 dans laquelle A⁻ représente un ion Cl⁻, Br⁻, I⁻ ou F⁻.

2. Matériau dentaire conforme à la revendication 1, qui contient de 0,1 à 1,5 % en poids, et de préférence de 0,1 à 0,6 % en poids, de l'agent de formule (I).

3. Matériau dentaire conforme à l'une des revendications précédentes, qui contient en outre au moins un monomère polymérisable à insaturation éthylénique, en tant que liant, et un amorceur de polymérisation radicalaire.

4. Matériau dentaire conforme à l'une des revendications précédentes, qui contient en outre une charge organique et/ou inorganique ou un mélange de charges.

5. Matériau dentaire conforme à la revendication 3, qui contient comme liant au moins un (méth)acrylate ou (méth)acrylamide, monofonctionnel ou polyfonctionnel, ou un mélange de tels composés.

6. Matériau dentaire conforme à la revendication 3 ou 5, qui contient comme liant un mélange de monomères acides et non acides.

7. Matériau dentaire conforme à l'une des revendications précédentes, qui contient en outre un solvant, de préférence de l'eau, du méthanol, de l'éthanol, du phénoxy-éthanol, de l'isopropanol, de l'acétate d'éthyle, de l'acétone, ou un mélange de deux de ces solvants ou plus.

8. Matériau dentaire conforme à l'une des revendications 1 à 3, 5 et 6, qui contient
- de 0,1 à 1,5 % en poids, et de préférence de 0,1 à 0,6 % en poids, d'agent de formule (I),
- de 20 à 95 % en poids, de préférence de 30 à 90 % en poids, et mieux encore de 35 à 80 % en poids de liant ;
- de 0,1 à 5 % en poids, de préférence de 0,3 à 4,0 % en poids, et mieux encore de 0,5 à 3,5 % en poids d'amorceur;
et en option,
- de 0 à 90 % en poids, de préférence de 2 à 85 % en poids, et mieux encore de 10 à 80 % en poids de charge;
et en option,
- de 0 à 80 % en poids, de préférence de 0 à 60 % en poids, et mieux encore de 0 à 40 % en poids de solvant.

9. Matériau dentaire conforme à l'une des revendications précédentes, pour utilisation en tant que matériau de plombage, ciment dentaire, matériau de scellement ou matériau de pansement temporaire.

10. Matériau dentaire conforme à l'une des revendications 1 à 8, pour utilisation en tant qu'adhésif dentinaire en masse fondue.

11. Matériau dentaire conforme à l'une des revendications 1 à 8, pour utilisation en tant que matériau de revêtement pour plombages, matériaux de restauration, prothèses dentaires et dents naturelles.

12. Matériau dentaire conforme à l'une des revendications 1 à 8, pour empêcher les dommages dentaires provoqués par *Streptococcus mutans* et/ou les lactobacilles.

13. Matériau dentaire conforme à l'une des revendications 1 à 8, pour empêcher les irritations dues au port d'une prothèse et provoquées par *Candida albicans.*

14. Utilisation d'un agent de formule (I) pour la préparation d'un matériau dentaire polymérisable conforme à la revendication 1.
